# DEMANDE DE BREVET EUROPEEN

(11) **EP 2 599 543 A1**
(43) Date de publication de la demande: **05.06.2013**
(21) Numéro de dépôt: 12290390.9
(22) Date de dépôt: 14.11.2012
(51) Int. Cl.: B01J 31/02

(54) **Composition catalytique contenant une fonction acide et procédé pour la dimérisation sélective de l'isobutène**

(30) Priorité: 30.11.2011 FR 1103653
(71) Demandeur: IFP Energies Nouvelles, 92852 Rueil Malmaison (FR)
(72) Inventeur: Magna, Lionel, 69007 Lyon (FR); Olivier-Bourbigou, Helene, 69230 Saint Genis-Laval (FR)

(57) **Abrégé**

Composition catalytique comprenant au moins un milieu liquide ionique non-aqueux de formule générale Q₁⁺A₁⁻, dans lequel Q₁⁺ représente un cation organique et A₁⁻ représente un anion, et au moins un composant ionique de formule générale Q₂⁺A₂⁻, dans laquelle Q₂⁺ représente un cation organique comprenant au moins une fonction acide sulfonique ou acide carboxylique, et A₂⁻ représente un anion. L'invention concerne également un procédé de dimérisation de l'isobutène utilisant la composition catalytique.

## Description

La présente invention concerne la dimérisation de l'isobutène. Plus particulièrement la présente invention a pour objet une nouvelle composition catalytique et un procédé de dimérisation de l'isobutène (pur ou en mélange avec d'autres hydrocarbures) utilisant cette nouvelle composition catalytique.

On sait que les dimères de l'isobutène (triméthyl-2,4,4-pentène-1 et -2) sont des intermédiaires intéressants pour la fabrication de différents produits ayant un intérêt commercial. A titre d'exemples, on peut citer les alcools supérieurs, les aldéhydes, les acides.

Le triméthyl-2,2,4-pentane peut être obtenu par hydrogénation des triméthylpentènes et constitue un additif recherché pour la reformulation des essences (absence de soufre, d'aromatique et d'oléfine, et faible volatilité s'ajoutent à un indice d'octane élevé : Indice d'Octane Moteur (RON) = Indice d'Octane Recherche (RON) = 100).

Ainsi, la dimérisation sélective de l'isobutène, suivie d'une hydrogénation des produits obtenus en triméthyl-2,2,4-pentane possédant un haut indice d'octane, constitue une voie intéressante qui permet
i) de remplacer le MTBE (Méthyl-Tert-Butyl-Ether : RON=118 ; MON=100), banni pour des raisons environnementales, et
ii) d'utiliser l'isobutène, issu des coupes C4 des procédés de craquage catalytique ou de craquage à la vapeur, matière première à la fabrication du MTBE.

La dimérisation (et aussi l'oligomérisation) de l'isobutène est une réaction exothermique catalysée par des acides. Différents acides ont été décrits dans la littérature tels que l'acide sulfurique, ou ses dérivés, les alumines chlorées ou fluorées, les zéolithes, les silices-alumines.... Cependant, les plus typiquement utilisés dans l'industrie sont l'acide phosphorique, généralement supporté, et les résines échangeuses d'ions (procédé SP-Isoéther licencié par Snamprogetti ou le procédé InAlk proposé par UOP).

La principale difficulté liée à ces procédés est l'obtention d'une bonne sélectivité en dimères. En effet, l'exothermicité de la réaction est souvent difficile à contrôler et entraîne la formation d'oligomères (essentiellement des oléfines en C12 et des oléfines en C16) obtenus par réactions parallèles à partir de l'isobutène. Ces oligomères ont des points d'ébullition trop élevés, et se trouvent en dehors, ou à la limite, des spécifications exigées pour les essences reformulées. Par ailleurs, ces oligomères contribuent à désactiver les catalyseurs.

Différents travaux dans la littérature décrivent certaines solutions pour minimiser la formation de ces oligomères.

Dans le cas des résines échangeuses d'ions (type Amberlyst-15 ou -35), l'utilisation d'un diluent (ou solvant) est souvent préconisée. La sélectivité en dimères dépend du choix de ce solvant. Les additifs les plus efficaces sont les alcools (US-A-5 877 372 ; US-A-4 100 220), qui conduisent à la co-production d'éthers, ou les éthers (dans US-A-4 447 668, le MTBE, l'ETBE, etc.). On peut citer les travaux de Snamprogetti (M. Marchionna and al. Catal. Today, 65 (2001) 397-403, GB 2 325 237) qui ont étudié l'influence de l'addition de MTBE ou de MeOH dans l'objectif de réutiliser les unités existantes de MTBE. Des sélectivités intéressantes en triméthylpentènes peuvent ainsi être atteintes mais à des conversions de l'isobutène souvent inférieures à 85 %.

La demande de brevet internationale WO-A-01/51 435 décrit un enchaînement de procédés dans lequel l'isobutène est produit par déhydratation de l'alcool *tert*-butylique. L'isobutène est dimérisé préférentiellement par une résine type Amberlyst A-15® en présence d'alcool tert-butylique (promoteur de sélectivité) et d'alcane (butane ou isobutane) comme diluant. La présence d'alcool encombré défavorise la formation d'éther mais aussi diminue la vitesse de réaction.

Tous les procédés décrits précédemment possèdent des limitations telles que les risques de désactivation prématurée du catalyseur par « encrassement » par les oligomères plus lourds ou encore la nécessité d'utiliser des additifs organiques pour maitriser la sélectivité de la réaction, additifs organiques souvent transformés et consommés lors de la réaction et donc non recyclables.

Les liquides ioniques non-aqueux de composition Q⁺A⁻ ont fait l'objet de plusieurs revues (par exemple, H. Olivier-Bourbigou et al., Appl. Catal. A: General, 2010, 1-56). Ils trouvent de nombreuses applications comme solvants pour la catalyse par les métaux de transition ou comme solvants d'extraction pour réaliser des extractions liquide-liquide.

La demande de brevet WO-A-00/16902 décrit l'utilisation d'un liquide ionique ne contenant pas d'acidité de Lewis obtenu par réaction d'un composé azoté (par exemple une amine ou un ammonium quaternaire) ou phosphoré avec un acide de Brønsted en quantités telles que le rapport dudit composé azoté ou phosphoré sur l'acide soit inférieur à 1. Ces milieux sont utilisés pour catalyser notamment l'alkylation du benzène avec le décène-1. Les acides de Brønsted utilisés sont ceux capables de générer des anions tels que BF₄⁻, PF₆⁻, RCOO⁻ ne contenant pas de fonction acide sulfonique ou acide carboxylique.

Il a également été décrit dans la demande FR2829039 que l'addition d'au moins un acide de Brønsted, noté HB, dans un milieu liquide non-aqueux à caractère ionique (milieu de type « sel fondu ») comprenant au moins un cation organique Q⁺ et un anion A⁻ et dans laquelle, lorsque A et B sont identiques, le rapport molaire de l'acide de Brønsted sur le liquide ionique est inférieur à 1/1, conduit à des compositions liquides qui peuvent être utilisées comme catalyseurs et solvants pour des réactions de catalyse acide. Dans cette demande de brevet, il était mentionné que la composition catalytique décrite pouvait être utilisée plus particulièrement dans la dimérisation de l'isobutène.

L'utilisation des liquides ioniques comme solvants et catalyseurs acides pour la dimérisation sélective de l'isobutène a aussi été décrite dans le brevet US7256152.

Les anions B de l'acide HB décrits dans FR2829039 et US7256152 sont choisis parmi les anions tétrafluoroborates, tétraalkylborates, hexafluorophosphates, hexafluoroantimonates, les alkylsulfonates et les arylsulfonates, les perfluoroalkylsulfonates, le fluorosulfonate, les sulfates, les phosphates, les perfluoroacétates, les perfluoroalkylsulfonamides, les fluorosulfonamides, les perfluoroalkylsulfométhides et les carboranes.

L'avantage de ces systèmes catalytiques liquides pour la réaction de dimérisation de l'isobutène en isooctènes est qu'ils sont peu miscibles avec les produits de la réaction, ceux-ci pouvant ainsi être séparés par décantation. La phase catalytique peut alors être recyclée et réutilisée, les consommations de catalyseur sont ainsi réduites. Cependant, ces systèmes possèdent encore des limitations. A l'instar des catalyseurs hétérogènes, il est parfois difficile de maintenir une recyclabilité du système catalytique tout en maintenant une bonne sélectivité en produits de dimérisation à haute conversion d'isobutène.

L'objet de la présente invention est de fournir une nouvelle composition catalytique permettant d'obtenir une très bonne recyclabilité du système catalytique sans baisse notable de son activité catalytique et sans baisse notable de sa sélectivité en dimères.

Plus particulièrement, la présente invention concerne une composition catalytique comprenant au moins un milieu liquide ionique non-aqueux de formule générale Q₁⁺A₁⁻, dans lequel Q₁⁺ représente un cation organique et A₁⁻ représente un anion, et au moins un composant ionique de formule générale Q₂⁺A₂⁻, dans laquelle Q₂⁺ représente un cation organique comprenant au moins une fonction acide sulfonique ou acide carboxylique, et A₂⁻ représente un anion.

Les composants ioniques sont définis comme étant des composés organiques acides susceptibles de donner au moins un proton.

Le liquide ionique Q₁⁺A₁⁻ est défini tel que Q₁⁺ représente un ammonium quaternaire et/ou un phosphonium quaternaire et/ou un trialkylsulfonium et A₁⁻ représente tout anion connu comme étant non-coordinant susceptible de former un sel liquide à basse température, c'est-à-dire en dessous de 150°C. Le composant ionique de formule générale Q₂⁺A₂⁻ est défini tel que Q₂⁺ représente un ammonium quaternaire et/ou un phosphonium quaternaire et/ou un trialkylsulfonium comprenant au moins une fonction acide sulfonique ou acide carboxylique et A₂⁻ représente tout anion connu comme étant non-coordinant susceptible de former un sel liquide à basse température, c'est-à-dire en dessous de 150°C.

Les anions A₁⁻ ou A₂⁻ considérés dans l'invention seront de préférence choisis parmi les anions tétrafluoroborate, tétraalkylborates, hexafluorophosphate, hexafluoroantimonate, les alkylsulfonates et les arylsulfonates (par exemple le méthylsulfonate ou le tosylate), les perfluoroalkylsulfonates (par exemple le trifluorométhylsulfonate), le fluorosulfonate, les sulfates, les phosphates, les perfluoroacétates (par exemple le trifluoroacétate), les perfluoroalkylsulfonamides (par exemple l'amidure de bis-trifluorométhanesulfonyle (N(CF₃SO₂)₂⁻), les fluorosulfonamides, les perfluoroalkylsulfométhides (par exemple le méthylure de tris-trifluorométhanesulfonyle (C(CF₃SO₂)₃⁻) et les carboranes, A₁⁻ et A₂⁻ étant identiques ou différents. De préférence, A₁⁻ et A₂⁻ sont identiques.

Les cations Q₁⁺ et Q₂⁺ considérés dans l'invention seront de préférence choisis parmi les ammoniums quaternaires et/ou les phosphoniums quaternaires et/ou les trialkylsulfoniums. La nature chimique de Q₁⁺ et Q₂⁺ peut être identique ou différente, elle est de préférence identique. On entend par nature chimique identique que si, par exemple, le cation Q₁⁺ est un ammonium quaternaire, Q₂⁺ est également un ammonium quaternaire. On entend par nature chimique différente que si, par exemple, le cation Q₁⁺ est un ammonium quaternaire, Q₂⁺ est choisi parmi un phosphonium quaternaire ou un trialkylsulfonium. Dans tous les cas, le cation organique Q₂⁺ de la composition Q₂⁺A₂⁻ contient au moins une fonction acide sulfonique ou acide carboxylique, alors que les substituants du cation organique Q₁⁺ n'ont pas de telle fonction. On entend par fonction acide sulfonique ou acide carboxylique un substituent hydrocarbyle ayant de 1 à 12 atomes de carbone contenant un groupement acide sulfonique (-SO₃H) ou acide carboxylique (-CO₂H) greffé sur le cation Q₂⁺.

Les ammoniums et/ou phosphoniums quaternaires de formule générale Q₁⁺ et Q₂⁺ répondent de préférence aux formules NR¹R²R³R⁴⁺ et PR¹R²R³R⁴⁺, ou aux formules générales R¹R²N=CR³R⁴⁺ et R¹R²P=CR³R⁴⁺ où
- pour Q₁⁺ : R¹, R², R³ et R⁴, identiques ou différents, représentent l'hydrogène, à l'exception du cation NH₄⁺ pour NR¹R²R³R⁴⁺, et de préférence un seul substituant peut représenter l'atome d'hydrogène, ou des restes hydrocarbyles ayant de 1 à 12 atomes de carbone, par exemple des groupements alkyles, saturés ou non saturés, cycloalkyles ou aromatiques, aryle ou aralkyle, comprenant de 1 à 12 atomes de carbone,
- et pour Q₂⁺ : au moins un substituent R¹, R², R³ ou R⁴ représente un reste hydrocarbyle ayant de 1 à 12 atomes de carbone contenant au moins une fonction acide sulfonique ou acide carboxylique, les substituants n'ayant pas de fonction acide sulfonique (-SO₃H) ou acide carboxylique (-CO₂H), identiques ou différents, sont définis comme précédemment pour Q₁⁺.

Les ammoniums et/ou phosphoniums quaternaires de formule générale Q₁⁺ et Q₂⁺ peuvent également être dérivés d'hétérocycles azotés (imidazolium, pyridinium, pyrrolidinium, pyrazolium, triazolium) ou phosphorés comportant 1, 2 ou 3 atomes d'azote et/ou de phosphore, de formules générales : 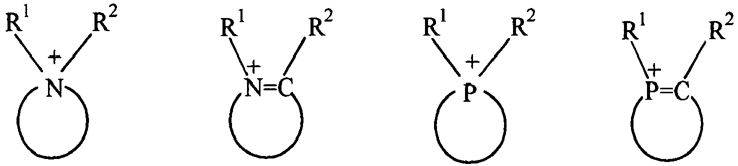 dans lesquelles les cycles sont constitués de 4 à 10 atomes, de préférence 5 à 6 atomes, R¹ et R² pour Q₁⁺ ou Q₂⁺ étant définis comme précédemment.

Les ammoniums et/ou phosphoniums quaternaires de formule générale Q₁⁺ et Q₂⁺ peut également consister en un cation répondant à l'une des formules générales :
R¹R²⁺N=CR³-R⁵-R³C=N⁺R¹R² et
R¹R²⁺P=CR³-R⁵-R³C=P⁺R¹R²
dans lesquelles R¹, R² et R³, identiques ou différents, sont définis comme précédemment pour Q₁⁺ ou Q₂⁺ et R⁵ représente un reste alkylène ou phénylène.

Parmi les groupements R¹, R², R³ et R⁴ on mentionnera les radicaux méthyle, éthyle, propyle, isopropyle, butyle, butyle secondaire, butyle tertiaire, amyle, phényle ou benzyle ; R⁵ pourra être un groupement méthylène, éthylène, propylène ou phénylène.

Parmi les groupements R¹, R², R³ et R⁴ contenant une fonction acide sulfonique ou acide carboxylique on mentionnera les radicaux -CH₂SO₃H, - (CH₂)₂SO₃H, -(CH₂)₃SO₃H, -(CH₂)₄SO₃H, -C(CH₃)₂-CH₂SO₃H, -CH₂-C(CH₃)₂SO₃H, -C(CH₃)H-CH₂SO₃H, -C₆H₄-CH₂SO₃H, -CH₂CO₂H, -(CH₂)₂CO₂H, -(CH₂)₃CO₂H, - (CH₂)₄CO₂H, -C(CH₃)₂-CH₂CO₂H, -CH₂-C(CH₃)₂CO₂H, -C(CF1₃)H-CH₂CO₂H, -C₆H₄-CH₂CO₂H.

Le cation ammonium et/ou phosphonium Q₁⁺ est choisi de préférence dans le groupe formé par le N-butylpyridinium, le N-éthylpyridinium, le 1-butyl-3-méthylimidazolium, le diéthylpyrazolium, le 1-éthyl-3-méthylimidazolium, le pyridinium, le triméthylphénylammonium, le tétrabutylphosphonium, le N-éthyl-N-méthylpyrrolidinium et le N-butyl-N-éthylpyrrolidinium.

Le cation ammonium et/ou phosphonium Q₂⁺ est choisi de préférence dans le groupe formé par le 1-butyl-3-(2-éthylsulfonique)imidazolium, le 1-éthyl-3-(2-éthylcarboxylique)imidazolium, le N-butyl-N-(2-éthylsulfonique)pyrrolidinium, le N-éthyl-N-(2-éthylcarboxylique)pyrrolidinium, la (2-éthylsulfonique)triéthylammonium et le triphényl(3-propylsulfonique)phosphonium.

Les trialkylsulfoniums de formule générale Q₁⁺ ou Q₂⁺ considérés dans l'invention ont pour formule générale SR¹R²R³⁺ dans laquelle
- pour Q₁⁺ : R¹, R² et R³, identiques ou différents, représentent des restes hydrocarbyles ayant de 1 à 12 atomes de carbone, par exemple des groupements alkyles ou alkényles, cycloalkyles ou aromatiques, aryles ou aralkyles, comprenant de 1 à 12 atomes de carbone,
- et pour Q₂⁺ : au moins un substituent R¹, R² ou R³ représente un reste hydrocarbyle ayant de 1 à 12 atomes de carbone contenant au moins une fonction acide sulfonique ou acide carboxylique, les substituants n'ayant pas de fonction acide sulfonique ou acide carboxylique, identiques ou différents, sont définis comme précédemment pour Q₁⁺.

A titre d'exemples des liquides ioniques utilisables Q₁⁺A₁⁻, on peut citer l'hexafluorophosphate de N-butylpyridinium, le tétrafluoroborate de N-éthyl pyridinium, l'hexafluoroantimonate de 1-butyl-3-méthylimidazolium, l'hexafluorophosphate de 1-butyl-3-méthylimidazolium, le trifluorométhylsulfonate de 1-butyl-3-méthylimidazolium, le fluorosulfonate de pyridinium, l'hexafluorophosphate de triméthylphénylammonium, le bis-trifluorométhylsulfonylamidure de 1-butyl-3-méthylimidazolium, le bis-trifluorométhylsulfonylamidure de N-ethyl-N-methylpyrrolidinium, le bis-trifluorométhylsulfonylamidure de triéthylsulfonium, le bis-trifluorométhylsulfonylamidure de tributylhexylammonium, le trifluoroacétate de 1-butyl-3-méthylimidazolium et le bis-trifluorométhylsulfonylamidure de 1-butyl-2,3-diméthylimidazolium.

Ces liquides ioniques peuvent être utilisés seuls ou en mélange. Ils ont une fonction de solvants.

A titre d'exemples des compositions Q₂⁺A₂⁻ utilisables, on peut citer le trifluorométhylsulfonate de 1-méthyl-3-(2-éthylsufonique)imidazolium, le bistriflylamidure de 1-éthyl-3-(2-éthylcarboxylique)imidazolium, le trifluorométhylsulfonate de N-butyl-N-(2-éthylsulfonique)pyrrolidinium, le bistriflylamidure de N-éthyl-N-(2-éthylcarboxylique)pyrrolidinium, le trifluorométhylsulfonate de (2-éthylsulfonique)triéthylammonium et le paratoluène sulfonate de triphenyl(3-propylsulfonique)phosphonium.

Ces compositions peuvent être utilisés seuls ou en mélange.

L'utilisation d'un composant ionique de formule Q₂⁺A₂⁻ tel que défini dans la composition catalytique de la présente invention permet de favoriser l'immobilisation de l'acide dans le liquide ionique par le greffage de la fonction acide (sulfonique ou carboxylique) sur le cation organique O₂⁺ composant de l'acide. C'est ainsi, que grâce à une structure chimique ressemblante, l'acide est parfaitement immobilisé dans le liquide ionique Q₁⁺A₁⁻ ce qui permet d'obtenir une excellente recyclabilité de la composition catalytique.

De façon préférée, dans la composition catalytique utilisée selon l'invention, les anions A₁⁻ et A₂⁻ sont identiques. Le rapport molaire entre le composant ionique Q₂⁺A₂⁻ et le liquide ionique Q₁⁺A₁⁻ est de préférence inférieur à 5/1, de préférence inférieur à 2/1.

Le liquide ionique Q₁⁺A₁⁻ permet de dissoudre le composant ionique Q₂⁺A₂⁻ qui en soit est très visqueux. On obtient ainsi un contrôle du niveau de l'acidité dans la composition catalytique ainsi qu'une mise en oeuvre d'un système biphasique en présence des réactifs et de leurs produits, tels que l'isobutène et ses dimères. Ceci permet une séparation facile (par décantation) et en conséquent une très bonne recyclabilité.

Les composés entrant dans la composition catalytique utilisée dans le procédé de l'invention peuvent être mélangés dans un ordre quelconque. Le mélange peut se faire par une simple mise en contact suivie d'une agitation jusqu'à formation d'un liquide homogène. Ce mélange peut être fait en dehors du réacteur utilisé pour l'application catalytique ou dans ce réacteur.

La présente invention a également comme objet un procédé de dimérisation d'isobutène mettant en jeu la composition catalytique. Le procédé de dimérisation selon l'invention s'applique à l'isobutène pur ou en mélange avec d'autres hydrocarbures.

Les provenances de l'isobutène sont diverses. Cependant, les plus courantes sont la déshydrogénation de l'isobutane, la déshydratation de l'alcool tert-butylique. L'isobutène peut aussi provenir d'une coupe C4 d'un craquage catalytique en lit fluidisé ou d'un craquage à la vapeur.

Dans ce dernier cas, l'isobutène peut être utilisé en mélange avec les n-butènes, l'isobutane et le butane. Le procédé selon l'invention présente alors l'avantage supplémentaire de permettre de convertir sélectivement l'isobutène sans qu'on ait à le séparer des autres constituants de la coupe. Un autre avantage du procédé selon l'invention est que la co-dimérisation isobutène-butène peut être limitée.

Des avancées récentes dans le domaine des biotechnologies montrent qu'il est également possible de générer de l'isobutène à partir du 2-méthyl-propanol (isobutanol), lui même obtenu à partir de sucres issues de la fermentation de la biomasse.

Le rapport volumique entre l'isobutène et la composition catalytique peut être compris entre 0,1/1 et 1000/1, de préférence entre 1/1 et 100/1. Il sera choisi de façon à obtenir les meilleures sélectivités.

Le procédé peut être conduit en système fermé, en système semi-ouvert ou en continu avec un ou plusieurs étages de réaction. A la sortie du réacteur, la phase organique contenant les produits de réaction est séparée.

Dans le procédé de dimérisation de l'invention, on peut ajouter à la composition catalytique un solvant organique comme un hydrocarbure aliphatique ou un hydrocarbure aromatique non ou partiellement miscible avec le liquide ionique qui permet une meilleure séparation des phases. On peut utiliser comme hydrocarbure aliphatique par exemple le pentane, l'heptane, le cyclohexane, le décane, le dodécane ou une charge paraffinique, seul ou en mélange. On peut utiliser comme hydrocarbure aromatique par exemple le toluène ou le xylène, seul ou en mélange.

La température à laquelle on effectue la réaction de dimérisation va par exemple de -50°C à 200°C ; elle est avantageusement inférieure à 100°C.

La réaction peut se faire à la pression autogène, celle-ci peut aussi être augmentée jusqu'à 10 MPa.

La réaction de dimérisation peut être conduite en utilisant une technique de distillation réactive.

Les produits obtenus par la présente invention peuvent être transformés ultérieurement selon différentes réactions, telles que hydrogénation, hydroformylation, oxydation, éthérification, époxydation ou hydratation.

Les exemples suivants illustrent l'invention sans en limiter la portée.

### Exemple 1 (selon l'invention) : Préparation de l'acide [MeIm(CH₂)₄SO₃H][CF₃SO₃]

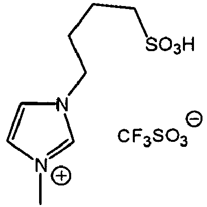

Dans un ballon de 250 mL on introduit sous atmosphère inerte 9,12 g (0,067 mmol) de 1,4-butanesultone que l'on dissout dans 150 mL de toluène. On additionne lentement 5,469 g (0,067 mmol) de 1-méthylimidazole. Le mélange est agité à température ambiante durant 1 h puis au reflux durant 12h. Après retour à température ambiante, le solide formé est filtré puis lavé au toluène et à l'éther diéthylique. Il est finalement séché sous vide à température ambiante (rendement >90%). Ce composé est mis en suspension dans 100 mL de toluène sec. On additione ensuite lentement 1 équivalent molaire d'acide triflique. Après 1 h de réaction à température ambiante, le toluène est éliminé sous vide dynamique. Le résidu liquide est enfin séché à 80°C sous vide dynamique. On obtient le [MeIm(CH₂)₄SO₃H][CF₃SO₃] avec un rendement quasi quantitatif. Les analyses RMN ¹H et ¹³C confirme la structure du produit attendu.

### Exemple 2 (selon l'invention) : Préparation du système catalytique liquide ionique [BMIm][CF₃SO₃] et acide [MeIm(CH₂)₄SO₃H][CF₃SO₃]

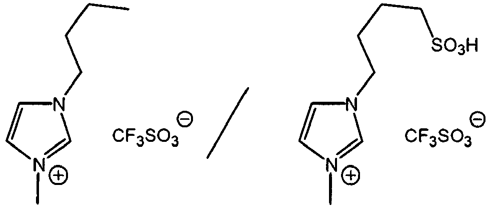

On a mélangé à température ambiante, sous atmosphère inerte, 4,37 g (0,012 mol) de [MeIm(CH₂)₄SO₃H][CF₃SO₃] décrit dans l'exemple 1 avec 2 mL de [BMIm][CF₃SO₃]. L'ensemble représente un volume de 5 mL. Le mélange est agité quelques minutes et conduit à une solution limpide contenant 2,4 mol/L d'acide.

### Exemples 3 (selon l'invention) : Dimérisation de l'isobutène utilisant la composition catalytique de l'Exemple 2

Dans un tube Fisher-Porter de volume 100 mL, muni d'un barreau magnétique et préalablement séché à l'étude et tiré sous vide, on introduit, sous atmosphère d'argon, la totalité du mélange préparé dans l'Exemple 2. Puis, on introduit, à température ambiante, 20 mL (11,2g) d'une charge liquide contenant 95 % d'isobutène et 5 % de n-butane. L'agitation est alors mise en route (temps zéro de la réaction). Après 20 minutes de réaction à 25°C, l'agitation est arrêtée. La phase organique surnageante est séparée de la phase liquide ionique et analysée par CPV (chromatographie en phase vapeur, avec l'heptane comme étalon externe) après traitement à la soude (10N) pour éliminer les éventuelles traces d'acide et séchage sur MgSO₄. La conversion de l'isobutène est de 71 %. La sélectivité en produit de dimérisation (C8) est de 86 %, la sélectivité en trimères (C12) est de 14 %, celle en tetramères (C16) moins que 1 %.

La phase liquide ionique contenant le système "[MeIm(CH₂)₄SO₃H][CF₃SO₃] / [BMIm][CF₃SO₃]" a été isolée et réutilisée sur plusieurs cycles de catalyse sans ajustement de la composition catalytique (exemples 4 à 13). Les résultats obtenus sont reportés dans le tableau.

**Tableau : Recyclage du système "[BMIm][CF₃SO₃]/[MeIm(CH₂)₄SO₃H][CF₃SO₃]"**

| Ex | Liquide ionique | Acide ajouté nature | mol | Conv. (% pds) | Sélectivités (% pds) | | |
|---|---|---|---|---|---|---|---|
| | | | | | C8 | C12 | C16 |
| | | | | | | | |
| 3 | [BMIm][CF₃SO₃] | [MeIm(CH₂)₄SO₃H] [CF₃SO₃] | 0.012 | 71 | 86 | 14 | < 1 |
| 4 | | Recyclage 1 | | 71 | 87 | 12 | < 1 |
| 5 | | Recyclage 2 | | 71 | 88 | 12 | < 1 |
| 6 | | Recyclage 3 | | 66 | 88 | 12 | < 1 |
| 7 | | Recyclage 4 | | 64 | 88 | 12 | < 1 |
| 8 | | Recyclage 5 | | 68 | 88 | 11 | <1 1 |
| 9 | | Recyclage 6 | | 60 | 89 | 10 | < 1 |
| 10 | | Recyclage 7 | | 67 | 89 | 11 | <1 1 |
| 11 | | Recyclage 8 | | 61 | 89 | 11 | <1 1 |
| 12 | | Recyclage 9 | | 66 | 90 | 10 | < 1 |
| 13 | | Recyclage 10 | | 68 | 90 | 10 | < 1 |

La comparaison des spectres **RMN** du proton de la phase liquide ionique avant et après cette série de recyclage démontre la parfaite immobilisation de l'acide dans le liquide ionique. La composition catalytique peut ainsi être recyclée sans baisse notable de son activité catalytique et sans baisse notable de sa sélectivité en dimères.

## Revendications

1. Composition catalytique comprenant au moins un milieu liquide ionique non-aqueux de formule générale Q₁⁺A₁⁻, dans lequel Q₁⁺ représente un cation organique et A₁⁻ représente un anion, et au moins un composant ionique de formule générale Q₂⁺A₂⁻, dans laquelle Q₂⁺ représente un cation organique comprenant au moins une fonction acide sulfonique ou acide carboxylique, et A₂⁻ représente un anion.

2. Composition catalytique selon la revendication 1, dans laquelle l'anion A₁⁻ ou A₂⁻ est choisi parmi les anions tétrafluoroborate, tétraalkylborates, hexafluorophosphate, hexafluoroantimonate, alkylsulfonates, arylsulfonates, perfluoroalkylsulfonates, fluorosulfonate, sulfates, phosphates, perfluoroacétates, perfluoroalkylsulfonamides, fluorosulfonamides, perfluoroalkylsulfométhides et carboranes, A₁⁻ et A₂⁻ étant identiques ou différents.

3. Composition catalytique selon la revendication 1 ou 2, dans laquelle Q₁⁺ représente un ammonium quaternaire et/ou un phosphonium quaternaire + et/ou un trialkylsulfonium, et Q₂⁺ représente un ammonium quaternaire et/ou un phosphonium quaternaire et/ou un trialkylsulfonium comprenant au moins une fonction acide sulfonique ou acide carboxylique, et A₁⁻ ou A₂⁻ représente tout anion connu comme étant non-coordinant et susceptible de former un sel liquide au-dessous de 150°C.

4. Composition catalytique selon la revendication 3, dans laquelle le cation ammonium et/ou phosphonium quaternaire Q₁⁺ ou Q₂⁺ est choisi parmi :
- les cations ammonium et/ou phosphonium quaternaires répondant à l'une des formules générales NR¹R²R³R⁴⁺ et PR¹R²R³R⁴⁺, ou à l'une des formules générales R¹R²N=CR³R⁴⁺ et R¹R²P=CR³R⁴⁺ où,
- pour Q₁⁺ : R¹, R², R³ et R⁴, identiques ou différents, représentent l'hydrogène, à l'exception du cation NH₄⁺ pour NR¹R²R³R⁴⁺, un seul substituant représentant l'atome d'hydrogène, ou des restes hydrocarbyles ayant de 1 à 12 atomes de carbone,
- et pour Q₂⁺ : au moins un substituent R¹, R², R³ ou R⁴ représente un reste hydrocarbyle ayant de 1 à 12 atomes de carbone contenant au moins une fonction acide sulfonique ou acide carboxylique, les substituants n'ayant pas de fonction acide sulfonique ou acide carboxylique, identiques ou différents, sont définis comme précédemment pour Q₁⁺,
- les cations ammonium et/ou phosphonium quaternaires dérivés d'hétérocycles azotés ou phosphorés comportant 1, 2 ou 3 atomes d'azote et/ou de phosphore, de formules générales : 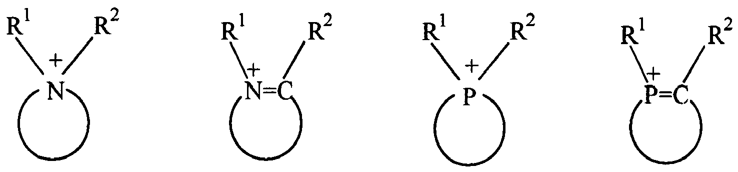 dans lesquelles les cycles sont constitués de 4 à 10 atomes, de préférence 5 à 6 atomes, R¹ et R² pour Q₁⁺ ou Q₂⁺ étant définis comme précédemment,
- les cations ammonium et/ou phosphonium quaternaires répondant à l'une des formules générales :
R¹R²⁺N=CR³-R⁵-R³C=N⁺R¹R²
R¹R²⁺P=CR³-R⁵-R³C=P⁺R¹R²
dans lesquelles R¹, R² et R³, identiques ou différents, sont définis comme précédemment pour Q₁⁺ ou Q₂⁺ et R⁵ représente un reste alkylène ou phénylène.

5. Composition catalytique selon la revendication 4, dans laquelle le cation ammonium et/ou phosphonium quaternaire Q₁⁺ est choisi dans le groupe formé par le N-butylpyridinium, le N-éthylpyridinium, le 1-butyl-3-méthylimidazolium, le diéthylpyrazolium, le 1-éthyl-3-méthylimidazolium, le pyridinium, le triméthylphénylammonium, le tétrabutylphosphonium, le N-éthyl-N-méthylpyrrolidinium et le N-butyl-N-éthylpyrrolidinium.

6. Composition catalytique selon la revendication 4, dans laquelle le cation ammonium et/ou phosphonium Q₂⁺ est choisi dans le groupe formé par le 1-butyl-3-(2-éthylsulfonique)imidazolium, le 1-éthyl-3-(2-éthylcarboxylique)-imidazolium, le N-butyl-N-(2-éthylsulfonique)pyrrolidinium, le N-éthyl-N-(2-éthylcarboxylique)pyrrolidinium, la (2-éthylsulfonique)triéthylammonium et le triphényl(3-propylsulfonique)phosphonium.

7. Composition catalytique selon la revendication 3, dans laquelle Q₁⁺ ou Q₂⁺ est un cation trialkylsulfonium répondant à la formule générale SR¹R²R³⁺ dans laquelle
- pour Q₁⁺ : R¹, R² et R³, identiques ou différents, représentent des restes hydrocarbyles ayant de 1 à 12 atomes de carbone,
- et pour Q₂⁺ : au moins un substituent R¹, R² ou R³ représente un reste hydrocarbyle ayant de 1 à 12 atomes de carbone contenant au moins une fonction acide sulfonique ou acide carboxylique, les substituants n'ayant pas de fonction acide sulfonique ou acide carboxylique, identiques ou différents, sont définis comme précédemment pour Q₁⁺.

8. Composition catalytique selon l'une des revendications précédentes, dans laquelle le liquide ionique Q₁⁺A₁⁻ est choisi parmi l'hexafluorophosphate de N-butylpyridinium, le tétrafluoroborate de N-éthyl pyridinium, l'hexafluoroantimonate de 1-butyl-3-méthylimidazolium, l'hexafluorophosphate de 1-butyl-3-méthylimidazolium, le trifluorométhylsulfonate de 1-butyl-3-méthylimidazolium, le fluorosulfonate de pyridinium, l'hexafluorophosphate de triméthylphénylammonium, le bis-trifluorométhylsulfonylamidure de 1-butyl-3-méthylimidazolium, le bis-trifluorométhylsulfonylamidure de N-ethyl-N-methylpyrrolidinium, le bis-trifluorométhylsulfonylamidure de triéthylsufonium, le bis-trifluorométhylsulfonylamidure de tributylhexylammonium, le trifluoroacétate de 1-butyl-3-méthylimidazolium et le bis-trifluorométhylsulfonylamidure de 1-butyl-2,3-diméthylimidazolium, seul ou en mélange.

9. Composition catalytique selon l'une des revendications précédentes, dans laquelle le composant ionique Q₂⁺A₂⁻ est choisi parmi le trifluorométhylsulfonate de 1-méthyl-3-(2-éthylsulfonique)imidazolium, le bistriflylamidure de 1-éthyl-3-(2-éthylcarboxylique)imidazolium, le trifluorométhylsulfonate de N-butyl-N-(2-éthylsulfonique)pyrrolidinium, le bistriflylamidure de N-éthyl-N-(2-éthylcarboxylique)pyrrolidinium, le trifluorométhylsulfonate de (2-éthylsulfonique)triéthylammonium et le paratoluène sulfonate de triphényl(3-propylsulfonique)phosphonium, seul ou en mélange.

10. Composition catalytique selon l'une des revendications précédentes, dans laquelle le rapport molaire entre le composant ionique Q₂⁺A₂⁻ et le liquide ionique Q₁⁺A₁⁻ est inférieur à 5/1.

11. Procédé de dimérisation de l'isobutène mettant en jeu une composition catalytique selon l'une des revendications 1 à 10.

12. Procédé de dimérisation de l'isobutène selon la revendication 11 dans lequel le rapport volumique entre l'isobutène et la composition catalytique est compris entre 0,1/1 et 1000/1.

13. Procédé de dimérisation de l'isobutène selon les revendications 11 ou 12 dans lequel on ajoute à la composition catalytique un hydrocarbure aliphatique ou un hydrocarbure aromatique non ou partiellement miscible avec le liquide ionique.

14. Procédé de dimérisation de l'isobutène selon les revendications 11 à 13 dans lequel on effectue la réaction de dimérisation à une température entre -50°C à 200°C, à une pression allant de la pression autogène à 10 MPa.

15. Procédé de dimérisation de l'isobutène selon les revendications 11 à 14 caractérisé en qu'il est conduit en système fermé, en système semi-ouvert ou en continu avec un ou plusieurs étages de réaction.
